(19) Europäisches Patentamt
European Patent Office
Office européen des brevets

(11) EP 3 623 373 A1

(12) EUROPEAN PATENT APPLICATION
published in accordance with Art. 153(4) EPC

(43) Date of publication:
**18.03.2020 Bulletin 2020/12**

(21) Application number: **18797555.2**

(22) Date of filing: **07.05.2018**

(51) Int Cl.:
***C07D 491/147*** *(2006.01)* ***A61K 31/444*** *(2006.01)*
***A61P 25/08*** *(2006.01)*

(86) International application number:
**PCT/JP2018/017577**

(87) International publication number:
**WO 2018/207715 (15.11.2018 Gazette 2018/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.05.2017 JP 2017093147**

(71) Applicant: **Eisai R&D Management Co., Ltd.**
**Tokyo 112-8088 (JP)**

(72) Inventors:
- **KUSHIDA Ikuo**
  **Tsukuba-shi**
  **Ibaraki 300-2635 (JP)**
- **ITO Yoko**
  **Tsukuba-shi**
  **Ibaraki 300-2635 (JP)**
- **MATSUDA Masaaki**
  **Tsukuba-shi**
  **Ibaraki 300-2635 (JP)**

(74) Representative: **Hoffmann Eitle**
**Patent- und Rechtsanwälte PartmbB**
**Arabellastraße 30**
**81925 München (DE)**

# (54) CRYSTALS OF PYRANODIPYRIDINE COMPOUND

(57) The present invention provides a crystal of the compound represented by formula (I) having applicability as a drug substance of pharmaceuticals.

[Chemical Formula 1]

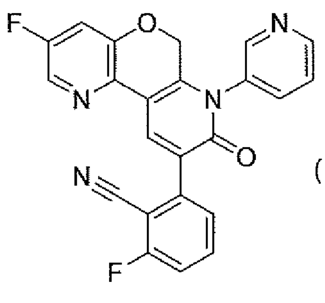

(I)

EP 3 623 373 A1

## Description

TECHNICAL FIELD

The present invention relates to a crystal of a pyranodipyridine compound having α-amino-3-hydroxy-5-methyl-4-isoxazolepropionic acid (AMPA) receptor inhibitory action.

BACKGROUND ART

It is known that glutamic acid released from the presynaptic region plays an important role in excitatory signaling in the central nervous system. This action is brought about when glutamic acid binds to glutamate receptors present in the postsynaptic region. The glutamate receptors are classified into ionotropic receptors and G-protein-coupled receptors, with the former being further classified into α-amino-3-hydroxy-5-methyl-4-isoxazolepropionic acid (AMPA) receptor, N-methyl-D-aspartic acid (NMDA) receptor, kainate receptor, and the like. Among the above, the AMPA receptor is a receptor that is widely expressed in the brain, and plays a key role in the regulation of fast excitatory synaptic transmission or synaptic plasticity.

Since the AMPA receptor thus plays a physiologically important role, its dysfunction is known to be involved in various diseases, for example, by causing abnormal excitability of neurons where the AMPA receptor is present. Examples of such diseases include epilepsy, various pains (peripheral nerve pain, central nerve pain, and nociceptive pain (each including chronic, acute, or intermittent pain)), various demyelinating diseases such as multiple sclerosis, various neurodegenerative diseases such as Parkinson's disease, Alzheimer's disease, amyotrophic lateral sclerosis (ALS), Huntington's chorea, and AIDS neuropathy, various psychiatric diseases such as anxiety, depression, bipolar mood disorder, dependency, drug abuse, and schizophrenia, motor dysfunction such as cerebral ischemia, head trauma, cerebrospinal injury, tremor, dystonia, and dyskinesia, and a developmental disorder such as autism. Inhibitors of the AMPA receptor (AMPA inhibitors) are therefore expected to lead to the treatment of these diseases, and are particularly known to be useful in treating epilepsy (Non Patent Literature 1).

Epilepsy is one of the most frequent central nerve diseases, and there are about 50 million or more epileptic patients worldwide. According to the World Health Organization, epilepsy is defined as "a chronic disorder of the brain caused by various causes; it is mainly characterized by recurrent seizures (epileptic seizures) derived from excessive electrical discharges in cerebral neurons, and the seizures involve manifestations of clinical and examination findings that vary greatly".

Examples of known epileptic seizures include partial seizures such as simple partial seizure, complex partial seizure, and secondary generalized seizure, absence seizure, myoclonic seizure, clonic seizure, tonic seizure, tonic-clonic seizure, atonic seizure, tuberous sclerosis complex, Dravet syndrome, progressive myoclonic epilepsy, Lafora disease, Unverricht-Lundborg disease, dentatorubral-pallidoluysian atrophy, fragile X syndrome, West syndrome, and Lennox-Gastaut syndrome. Epilepsy treatment is based on pharmacotherapy with anti-epileptic drugs. The goal of epilepsy treatment is to eliminate epileptic seizures, and avoid the development of side effects caused by the treatment. Treatment with anti-epileptic drugs begins with a single drug in principle. Generally, single-drug treatment is carried out by sequentially using two or three different drugs, and if this is not successful, multiple drug treatment is attempted. Amelioration of seizures through the treatment with anti-epileptic drugs can be expected in about 70% of patients with new onset of epilepsy. It is known, however, that in the remaining about 30% of patients, epileptic seizures are difficult to suppress even with drug treatment including multiple drug therapy.

An epileptic seizure is believed to be caused when abnormal excitability of some neurons develops into abnormal synchronization of firing in an entire population of neurons, and there have been many reports that glutamate neurons, in particular, the AMPA receptor, play a key role in the occurrence and propagation of an epileptic seizure. For example, it has been reported that AMPA inhibitors suppress the occurrence and propagation of convulsions in rat bicuculline-induced convulsion models (Non Patent Literatures 2 and 3); additionally, it is well known that AMPA inhibitors exhibit potent anticonvulsant action in a wide range of convulsion models (Non Patent Literatures 4 and 5). Further, it is known that AMPA inhibitors also have seizure-stopping action in status epilepticus models that experience extremely serious and continuous seizures, and thus, AMPA inhibitors are also expected to be applied to status epilepticus (Non Patent Literature 6).

It has been reported that similarly in humans, the expression of the AMPA receptor was increased in hippocampal neurons containing an epileptic focus, which were sampled from epileptic patients (Non Patent Literature 7). Further, because AMPA receptor inhibitors have been reported to have anticonvulsant action in humans, they are expected to have efficacy particularly as agents for treating epilepsy (Non Patent Literature 5).

As described above, AMPA inhibitors are expected to become therapeutic drugs for various central nerve diseases such as epilepsy; however, they are known to cause central nervous system depressant action such as sedation or loss of coordination, at a dose substantially equivalent to or lower than that showing main effect (Non Patent Literature

8). It has been reported that in humans, the central nervous system depressant action is reduced if the dose is gradually increased (Non Patent Literature 9); however, central nervous system depressant action is observed at a middle to high dose, which has become a problem that lowers the quality of life of epileptic patients in need of long-term administration, and also leads to the restriction of the dose.

The following compound is known as a compound having AMPA receptor inhibitory action (Patent Literature 1):

[Chemical Formula 1]

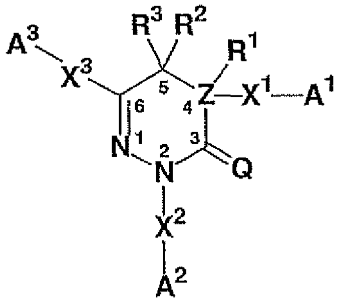

wherein $A^1$, $A^2$, and $A^3$ may each independently be a C6-14 aromatic hydrocarbon cyclic group or 5- to 14-membered aromatic heterocyclic group; $X^1$, $X^2$, and $X^3$ may each independently be a single bond; Q may be an oxygen atom; Z may be a carbon atom; $R^1$ and $R^2$ may be attached to each other such that $CR^2$-$ZR^1$ forms a carbon-carbon double bond represented by C=C; and $R^3$ may be attached to any atom on $A^3$, and together with the atom, may form an optionally substituted 5- to 8-membered heterocyclic ring.

In particular, Patent Literature 1 discloses as Example 9 a compound having a tricyclic skeleton represented by the following formula:

[Chemical Formula 2]

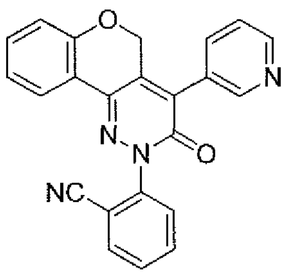

Citation List

Patent Literature

[Patent Literature 1] WO 02/22587

Non Patent Literature

[Non Patent Literature 1] Daniela Catarzi et al. Medicinal Research Reviews, vol. 27, No. 2, pp. 239-278, 2007
[Non Patent Literature 2] Rogawski MA et al. Acta Neurol Scand Suppl. 2013; (197): 9-18
[Non Patent Literature 3] Alfonso Tortorella et al. JPET 280: 1401-1405, 1997
[Non Patent Literature 4] De Sarro et al. Curr Top Med Chem. 2005; 5(1): 31-42
[Non Patent Literature 5] Russo E et al. Expert Opin Investig Drugs. 2012 Sep; 21(9): 1371-89
[Non Patent Literature 6] Brita Fritsch et al. Epilepsia, 51(1): 108-117, 2010
[Non Patent Literature 7] Hosford DA et al. J Neurosci 1991; 11: 428-434 [Non Patent Literature 8] Shun-ichi Yamaguchi et al. Epilepsy Research, 15 (1993) 179-184
[Non Patent Literature 9] Hanada T. Expert Opin Drug Discov. 2014 Feb 24, 9(4): 449-458

SUMMARY OF THE INVENTION

TECHNICAL PROBLEM

The compound represented by the following formula (I) (2-fluoro-6-(3-fluoro-8-oxo-7-(pyridin-3-yl)-7,8-dihydro-6H-pyrano[3,2-b:5,4-b']dipyridin-9-yl)benzonitrile, hereinafter also referred to as "compound (I)") has a potential use as an agent for treating epilepsy, which has AMPA receptor inhibitory action, and reduced central nervous system depressant action.

[Chemical Formula 3]

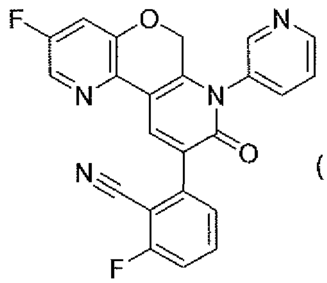

(I)

In general, the physical properties of the compound and the crystal used as pharmaceuticals have a great influence on the drug bioavailability, the purity of the drug substance, the formulations of the drug, or the like. Accordingly, an object of the present invention is to provide a crystal of compound (I).

SOLUTION TO PROBLEM

In view of the above, the present inventors conducted extensive research on compound (I), and as the result, the inventors have found a crystal of compound (I), and completed the present invention.
In summary, the present invention relates to <1> to <18> set forth below.

<1> A crystal of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

[Chemical Formula 4]

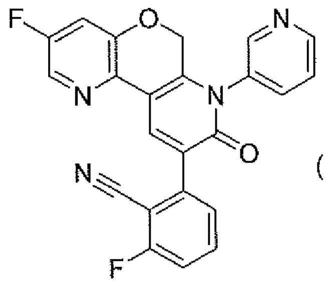

(I)

<2> The crystal according to <1>, wherein the crystal is a Type 1 crystal of an anhydrate(i.e. "Type 1 anhydrate crystal") of 2-fluoro-6-(3-fluoro-8-oxo-7-(pyridin-3-yl)-7,8-dihydro-6H-pyrano[3,2-b:5,4-b']dipyridin-9-yl)benzonitrile, having a diffraction peak at a diffraction angle (2θ ± 0.2°) of 10.8° in powder X-ray diffraction.
<3> The crystal according to <2>, wherein the crystal has diffraction peaks at diffraction angles (2θ ± 0.2°) of 10.2°, 10.8° and 30.4° in powder X-ray diffraction.
<3.1> The crystal according to <2>, wherein the crystal has diffraction peaks at diffraction angles (2θ ± 0.2°) of 10.2°, 10.8°, 16.8°, 26.2° and 30.4° in powder X-ray diffraction.
<3.2> The crystal according to <2>, wherein the crystal has diffraction peaks at diffraction angles (2θ ± 0.2°) of 10.2°, 10.8°, 16.8°, 18.7°, 23.4°, 24.9°, 26.2°, 27.1°, 28.1° and 30.4° in powder X-ray diffraction.
<4> The crystal according to <1>, wherein the crystal is a Type 1 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having peaks at chemical shifts (δ ± 0.5 ppm) of 65.8 ppm, 107.2 ppm, 113.4 ppm, 126.4 ppm, 145.1 ppm and 160.1 ppm in $^{13}C$ solid state NMR

spectroscopy.

<5> The crystal according to <1>, wherein the crystal is a Type 1 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a powder X-ray diffraction pattern shown in Figure 1.

<6> The crystal according to <1>, wherein the crystal is a Type 3 crystal of an anhydrate(i.e. "Type 3 anhydrate crystal") of 2-fluoro-6-(3-fluoro-8-oxo-7-(pyridin-3-yl)-7,8-dihydro-6H-pyrano[3,2-b:5,4-b']dipyridin-9-yl)benzonitrile, having a diffraction peak at a diffraction angle (2θ ± 0.2°) of 24.4° in powder X-ray diffraction.

<7> The crystal according to <6>, wherein the crystal has diffraction peaks at diffraction angles (2θ ± 0.2°) of 15.8°, 18.4° and 24.4° in powder X-ray diffraction.

<7.1> The crystal according to <6>, wherein the crystal has diffraction peaks at diffraction angles (2θ ± 0.2°) of 13.7°, 15.8°, 18.4° and 24.4° in powder X-ray diffraction.

<7.2> The crystal according to <6>, wherein the crystal has diffraction peaks at diffraction angles (20 ± 0.2°) of 11.8°, 13.7°, 15.8°, 17.5°, 18.4°, 19.3°, 21.1°, 23.6°, 24.4° and 26.0° in powder X-ray diffraction.

<8> The crystal according to <1>, wherein the crystal is a Type 3 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having peaks at chemical shifts (δ ± 0.5 ppm) of 64.3 ppm, 110.4 ppm, 122.7 ppm, and 127.1 ppm in $^{13}C$ solid state NMR spectroscopy.

<9> The crystal according to <1>, wherein the crystal is a Type 3 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a powder X-ray diffraction pattern shown in Figure 3.

<10> The crystal according to <1>, wherein the crystal is a crystal of a hydrate(i.e. "Hydrate crystal") of 2-fluoro-6-(3-fluoro-8-oxo-7-(pyridin-3-yl)-7,8-dihydro-6H-pyrano[3,2-b:5,4-b']dipyridin-9-yl)benzonitrile, having a diffraction peak at a diffraction angle (2θ ± 0.2°) of 5.5° in powder X-ray diffraction.

<11> The crystal according to <10>, wherein the crystal has diffraction peaks at diffraction angles (2θ ± 0.2°) of 5.5°, 14.0° and 27.2° in powder X-ray diffraction.

<11.1> The crystal according to <10>, wherein the crystal has diffraction peaks at diffraction angles (2θ ± 0.2°) of 5.5°, 8.5°, 14.0°, 15.4°, 16.1°, 17.0°, 22.6°, 26.5° and 27.2° in powder X-ray diffraction.

<12> The crystal according to <1>, wherein the crystal is a crystal of a hydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having peaks at chemical shifts (δ ± 0.5 ppm) of 63.7 ppm, 100.4 ppm, 109.6 ppm, 117.2 ppm, 129.1 ppm, 147.3 ppm and 156.3 ppm in $^{13}C$ solid state NMR spectroscopy.

<13> The crystal according to <1>, wherein the crystal is a crystal of a hydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a powder X-ray diffraction pattern shown in Figure 5.

<14> A pharmaceutical composition comprising the crystal according to any one of <1> to <13> as an active ingredient.

<15> The pharmaceutical composition according to <14>, wherein the pharmaceutical composition is an AMPA receptor inhibitor.

<16> The pharmaceutical composition according to <14>, for treating epilepsy.

<17> An agent for treating epilepsy, comprising the crystal according to any one of <1> to <13>.

<18> A method for treating epilepsy, comprising administering to a patient the crystal according to any one of <1> to <13>.

## ADVANTAGEOUS EFFECTS OF INVENTION

According to the present invention, it is possible to provide a crystal of compound (I) which has good physical properties and expected to have applicability as a drug substance of pharmaceuticals.

## BRIEF DESCRIPTION OF DRAWINGS

[Figure 1] Figure 1 shows a powder X-ray diffraction pattern of a Type 1 anhydrate crystal of compound (I) obtained in Example 1. The abscissa shows the diffraction angle (2θ), and the ordinate shows the peak intensity.

[Figure 2] Figure 2 shows a $^{13}C$ solid state NMR spectrum of the Type 1 anhydrate crystal of compound (I) obtained in Example 1. The abscissa shows the chemical shifts (δ), and the ordinate shows the peak intensity.

[Figure 3] Figure 3 shows a powder X-ray diffraction pattern of a Type 3 anhydrate crystal of compound (I) obtained in Example 2. The abscissa shows the diffraction angle (2θ), and the ordinate shows the peak intensity.

[Figure 4] Figure 4 shows a $^{13}C$ solid state NMR spectrum of the Type 3 anhydrate crystal of compound (I) obtained

in Example 2. The abscissa shows the chemical shifts (δ), and the ordinate shows the peak intensity.

[Figure 5] Figure 5 shows a powder X-ray diffraction pattern of a Hydrate crystal of compound (I) obtained in Example 3. The abscissa shows the diffraction angle (2θ), and the ordinate shows the peak intensity.

[Figure 6] Figure 6 shows a $^{13}C$ solid state NMR spectrum of the Hydrate crystal of compound (I) obtained in Example 3. The abscissa shows the chemical shifts (δ), and the ordinate shows the peak intensity.

[Figure 7] Figure 7 is a thermal analysis TG-DTA chart of the Hydrate crystal of compound (I) obtained in Example 3. The abscissa shows the temperature, the left ordinate shows the weight change of TG, and the right ordinate shows the heat flow of DTA.

## DESCRIPTION OF EMBODIMENTS

Hereinafter, the crystal of compound (I) and a production method thereof of the present invention will be described in detail.

As used herein, the term "crystal" refers to a crystal of an anhydrate or hydrate of compound (I).

Preferred crystals of compound (I) herein include:

a crystal of an anhydrate of compound (I), having a diffraction peak at a diffraction angle (2θ ± 0.2°) of 10.8° in powder X-ray diffraction (Type 1 anhydrate crystal);

a crystal of an anhydrate of compound (I), having diffraction peaks at diffraction angles (2θ ± 0.2°) of 10.2°, 10.8° and 30.4° in powder X-ray diffraction (Type 1 anhydrate crystal);

a crystal of an anhydrate of compound (I), having diffraction peaks at diffraction angles (2θ ± 0.2°) of 10.2°, 10.8°, 16.8°, 26.2° and 30.4° in powder X-ray diffraction (Type 1 anhydrate crystal);

a crystal of an anhydrate of compound (I), having peaks at chemical shifts (δ ± 0.5 ppm) of 65.8 ppm, 107.2 ppm, 113.4 ppm, 126.4 ppm, 145.1 ppm and 160.1 ppm in $^{13}C$ solid state NMR spectroscopy (Type 1 anhydrate crystal);

a crystal of an anhydrate of compound (I), having a diffraction peak at a diffraction angle (2θ ± 0.2°) of 24.4° in powder X-ray diffraction (Type 3 anhydrate crystal);

a crystal of an anhydrate of compound (I), having diffraction peaks at diffraction angles (2θ ± 0.2°) of 15.8°, 18.4° and 24.4° in powder X-ray diffraction (Type 3 anhydrate crystal);

a crystal of an anhydrate of compound (I), having diffraction peaks at diffraction angles (2θ ± 0.2°) of 13.7°, 15.8°, 18.4° and 24.4° in powder X-ray diffraction (Type 3 anhydrate crystal);

a crystal of an anhydrate of compound (I), having peaks at chemical shifts (δ ± 0.5 ppm) of 64.3 ppm, 110.4 ppm, 122.7 ppm and 127.1 ppm in $^{13}C$ solid state NMR spectroscopy (Type 3 anhydrate crystal);

a crystal of a hydrate of compound (I), having diffraction peaks at diffraction angles (2θ ± 0.2°) of 5.5° in powder X-ray diffraction (Hydrate crystal);

a crystal of a hydrate of compound (I), having diffraction peaks at diffraction angles (2θ ± 0.2°) of 5.5°, 14.0° and 27.2° in powder X-ray diffraction (Hydrate crystal); and

a crystal of a hydrate of compound (I), having peaks at chemical shifts (δ ± 0.5 ppm) of 63.7 ppm, 100.4 ppm, 109.6 ppm, 117.2 ppm, 129.1 ppm, 147.3 ppm and 156.3 ppm in $^{13}C$ solid state NMR spectroscopy (Hydrate crystal).

The diffraction peaks in powder X-ray diffraction and chemical shifts in $^{13}C$ solid state NMR spectroscopy described above are specific to each of the Type 1 anhydrate crystal of compound (I), Type 3 anhydrate crystal of compound (I), and Hydrate crystal of compound (I), and thus, they are peaks characteristic to each of the crystals.

In general, an error may be generated within the range of ±0.2° in diffraction angles (2θ) in powder X-ray diffraction. Thus, it needs to be understood that each value of the diffraction angle described above includes numerical values within the range of about ±0.2° in addition to the above-described value. Thus, with regard to certain compounds, not only a crystal which has peaks in powder X-ray diffraction at the diffraction angles completely identical to the above-described values, but also a crystal which has peaks in powder X-ray diffraction at the diffraction angles identical to the above-described values with an error within a range of about ±0.2° is included in the present invention as the same crystal.

For example, the phrase "having a diffraction peak at a diffraction angle (2θ ± 0.2°) of 10.8°" herein means "having a diffraction peak at a diffraction angle (2θ) of 10.6° to 11.0°", and the same applies for other diffraction angles.

In general, the peak intensity or half width of the diffraction angle (2θ) in powder X-ray diffraction is different for each measurement according to the difference in measurement conditions or variation of the size or shape of each particle of the powder crystal used as a measurement sample, even when the crystal forms are the same. Thus, the obtained peak intensity or half width for each measurement is not always constant. Therefore, in a comparison of powder X-ray diffraction pattern, even when there is a difference in the peak intensity or half width at the same diffraction angle (2θ), the difference does not mean that the crystals are different in crystalline form. Thus, when a crystal has a powder X-ray diffraction pattern with such a difference to the characteristic diffraction peaks of the specific crystal of the present invention, it means that the crystal is in the same crystalline form as the crystal of the present invention. Furthermore,

the phrase "having an X-ray powder diffraction pattern of Figure 1" herein means that, not only a crystal having an X-ray powder diffraction pattern perfectly identical to the powder X-ray diffraction pattern shown in Figure 1, but also a crystal having a powder X-ray diffraction pattern identical to the pattern shown in Figure 1 in characteristic diffraction angles but different in peak intensity or half width are all the same as the crystal of the present invention.

As used herein, the phrase "chemical shifts ($\delta \pm 0.5$ ppm) of 65.8 ppm, 107.2 ppm, 113.4 ppm, 126.4 ppm, 145.1 ppm and 160.1 ppm" means that "$^{13}C$ solid state NMR spectroscopy is performed under the normal measurement conditions or substantially the same condition as that described in the specification, and in either case, the crystal having substantially equivalent peaks at the chemical shifts ($\delta \pm 0.5$ ppm) of 65.8 ppm, 107.2 ppm, 113.4 ppm, 126.4 ppm, 145.1 ppm and 160.1 ppm is obtained".

In general, when determining whether or not the crystal "having substantially equivalent peaks", an error may be generated within the range of $\pm 0.5$ ppm in chemical shifts in $^{13}C$ solid state NMR spectroscopy. Thus, it needs to be understood that each value of the chemical shifts described above also includes numerical values within the range of about $\pm 0.5$ ppm in addition to the described value. Thus, not only a crystal which is completely identical in the chemical shifts in $^{13}C$ solid state NMR spectroscopy, but also a crystal which is identical in the chemical shifts with an error within the range of about $\pm 0.5$ ppm is included in the present invention as the same crystal. Therefore, for example, the phrase "having a peak at a chemical shift ($\delta \pm 0.5$ ppm) of 65.8 ppm" herein means "having a peak in the range of chemical shifts ($\delta$) of 65.3 ppm to 66.3 ppm", and the same applies for other chemical shifts in $^{13}C$ solid state NMR spectroscopy.

Hereinafter, a method for producing a crystal of compound (I), which is an embodiment of the present invention, will be described.

Method for Producing Compound (I)

Compound (I) may be produced by a method well known to those skilled in the art. For example, compound (I) can be synthesized by the method described in the Reference Example described below.

Method for Producing Crystal of Compound (I)

Hereinafter, a method for producing a crystal of compound (I) is described. The crystal of compound (I) can be produced by heating and dissolving compound (I) in a solvent, and then cooling the dissolved compound (I) under stirring to crystallize compound (I). For example, the Type 1 and Type 3 crystals of an anhydrate of compound (I) and the crystal of a hydrate of compound (I) can be produced by appropriately modifying the methods described in Examples.

Compound (I) used in the crystallization may be in any form. For example, compound (I) may be in a form of a solvate, hydrate or anhydrate, and an amorphous or a crystal (including a crystal made of a plurality of polymorphs), and may be a mixture thereof.

Examples of the solvent used for crystallization include alcohol solvents such as methanol, ethanol, 2-propanol, 1-propanol, and 1-butanol; acetonitrile; amide solvents such as N,N-dimethylformamide; ester solvents such as ethyl acetate; saturated hydrocarbon solvents such as hexane or heptane; ketone solvents such as acetone or 2-butanone; and ether solvents such as t-butyl methyl ether; and water. These solvents may be used alone or may be used in combination of two or more. The crystallization with a water-containing solvent such as a solvent mixture of ethanol and water affords a crystal of a hydrate of compound (I).

The use amount of the solvent can be appropriately selected, with an amount which allows compound (I) to be dissolved by heating or allows the suspension to be stirrable, as a lower limit, and with an amount which does not significantly reduce the yield of crystal, as an upper limit.

In the crystallization, a desired crystal of compound (I) may be added as a seed crystal. The temperature of the solution when adding the seed crystal is not particularly limited, but preferably 0 to 80°C.

The temperature when heating and dissolving compound (I) may be determined by appropriately selecting the temperature to dissolve compound (I), depending on the solvent. It is preferred that the temperature is preferably in the range from 50°C to the temperature at which the solvent initiates reflux, and more preferably 55 to 80°C.

In the cooling during the crystallization, a rapid cooling may provide a crystal containing various forms of crystals (polymorph). Thus, it is preferred that the cooling is preferably performed while adjusting the appropriate cooling rate in consideration of the influence on the crystal such as quality or grain size. Examples of the preferred cooling rate include 5 to 40°C/hour.

The final crystallization temperature may be appropriately selected considering, for example, the yield or quality of the crystals, but it is preferably -25 to 30°C.

The crystal obtained after crystallization can be separated by normal filtration to afford a crystal of interest. The filtered crystal may be optionally washed with a solvent, and may be further dried. The solvent used for washing the crystal may be the same as the solvent for crystallization. Preferred examples of the solvent include ethanol, acetone, 2-butanone, ethyl acetate, diethyl ether, t-butyl methyl ether, and hexane. These solvents may be used alone or may

be used in combination of two or more.

The crystal separated by the filtration may be dried, as appropriate, by leaving under the atmosphere or under a nitrogen stream, or by heating.

The drying time may be determined by appropriately selecting the time until the amount of the residual solvent falls below a predetermined quantity, depending on the production volume, the drying apparatus, the drying temperature, or the like. Moreover, the drying may be carried out under ventilation or under reduced pressure. The degree of the reduced pressure may be appropriately selected depending on the production volume, drying apparatus, drying temperature, or the like. After drying, the resulting crystal may be leaved in the atmosphere as required.

The crystal of compound (I) obtained by the production method described above, as shown in the activity data in the Pharmacological Test Example described below, have AMPA receptor inhibitory action, and hence, have applicability as agents for treating epilepsy.

[Pharmaceutical Composition]

Another embodiment of the present invention is a pharmaceutical composition comprising the crystal of compound (I) and a pharmaceutically acceptable additive. The pharmaceutical composition of the invention can be produced by mixing a pharmaceutically acceptable additive with the crystal of compound (I). A pharmaceutical composition of the invention could be produced according to the known method such as a method described in the General Rules for Preparations of the Japanese Pharmacopoeia 16th Edition.

A pharmaceutical composition of the embodiment could be administered to patients appropriately depending on the dosage form.

The dose of compound (I) according to the present invention will vary depending on the severity of the condition, age, sex, body weight, type of the dosage form or salt, specific type of the disease, and the like; generally, however, for an adult, in the case of oral administration, the daily dose is about 30 μg to 10 g, preferably 100 μg to 5 g, and more preferably 100 μg to 1 g, and in the case of administration by injection, the daily dose is about 30 μg to 1 g, preferably 100 μg to 500 mg, and more preferably 100 μg to 300 mg, each administered in single or several divided doses.

EXAMPLES

The crystal of compound (I) of the present invention can be produced, for example, using the methods described in the following Examples, and the effects of the compounds can be confirmed using the methods described in the following Test Examples. It should be noted, however, that these examples are illustrative, and the present invention is in any case not limited to the following specific examples, and modifications may be made thereto without departing from the scope of the present invention.

In the following examples, the powder X-ray crystal diffraction of the produced crystals was carried out by placing the produced crystal on a sample stage of a powder X-ray diffractometer, and analyzing the crystal under the following conditions.

(Measurement Conditions)

Sample holder: aluminum holder
Target: copper
Detector: scintillation counter
Tube voltage: 50 kV
Tube current: 300 mA
Slit: Divergence slit 0.5 mm, Scattering slit open, Light receiving slit open Scanning rate: 5°/minute
Sampling interval: 0.02°
Scanning range: 5 to 35°

The $^{13}C$ solid state NMR spectra of the crystals were measured under the following conditions.

(Measurement Conditions)

Used equipment: Avance 400 MHz (manufactured by BRUKER Co., Ltd.) 7 mm-CPMAS probe (manufactured by BRUKER Co., Ltd.)
Measurement nucleus: $^{13}C$ (100.6248425 MHz)
Measurement temperature: room temperature (22°C)
Pulse mode: CPTOSS measurement (spinning side band elimination method)
Rotation frequency: 5000 Hz

Pulse repetition time: 60 sec
Contact time: 1 msec
Cumulative number: 2400 times
Reference material: glycine (external reference: 176.03 ppm)

Compounds for which document names or the like are noted were produced in accordance with the document or the like.

The abbreviations used herein are conventional abbreviations well known to those skilled in the art. The following abbreviations will be used herein:

$(Ataphos)_2PdCl_2$: bis(di-t-butyl(4-dimethylaminophenyl)phosphine)dichloropalladium (II)
DCM: dichloromethane
DMF: N,N-dimethylformamide
DMSO: dimethylsulfoxide
n-: normal
NBS: N-bromosuccinimide
THF: tetrahydrofuran
$^1$H-NMR: proton nuclear magnetic resonance spectrometry
MS: mass spectrometry

In the following Examples and Reference Examples, the "room temperature" generally refers to about 10°C to about 35°C. "%" refers to percent by weight, unless otherwise specified.

The chemical shifts in proton nuclear magnetic resonance spectroscopy are recorded in $\delta$ units (ppm) relative to tetramethylsilane, and coupling constants are recorded in hertz (Hz). Abbreviations for splitting patterns are as follows: s: singlet, d: doublet, t: triplet, q: quartet, m: multiplet, br.s: broad singlet.

For reactions using a microwave reactor in the reference examples, initiator™ or initiator+™ from Biotage Corporation was used.

For chromatography, as the silica gel, Silica Gel60 (70-230 mesh ASTM) from Merck Corporation or PSQ60B from Fuji Silysia Chemical Ltd. was used, or a pre-packed column {column: Hi-Flash™ Column (Silicagel) from Yamazen Corporation, size: any of S (16 x 60 mm), M (20 x 75 mm), L (26 x 100 mm), 2L (26 x 150 mm), and 3L (46 x 130 mm); or Biotage™ SNAP Ultra Silica Cartridge from Biotage Corporation, size: any of 10 g, 25 g, and 50 g} was used.

As the NH silica gel, CHROMATOREX NH-DM2035 from Fuji Silysia Chemical Ltd. was used, or a pre-packed column {column: Hi-Flash™ Column (Amino) from Yamazen Corporation, size: any of S (16 x 60 mm), M (20 x 75 mm), L (26 x 100 mm), 2L (26 x 150 mm), and 3L (46 x 130 mm); or Presep™ (Luer Lock) $NH_2$(HC) from Wako Pure Chemical Industries, Ltd., size: any of type M (14 g/25 mL), type L (34 g/70 mL), type 2L (50 g/100 mL), and type 3L (110 g/200 mL)} was used.

As neutral alumina, aluminium oxide 90 active neutral, 70-230 mesh, Merck, E6NXX was used.

As the names of the compounds shown below, those displayed on the "E-Notebook" version 12 (PerkinElmer Co., Ltd.) were used, except for commonly used reagents.

Reference Example 1

Synthesis of 2-fluoro-6-(3-fluoro-8-oxo-7-(pyridin-3-yl)-7,8-dihydro-6H-pyrano[3,2-b:5,4-b']dipyridin-9-yl)benzonitrile

[Chemical Formula 5]

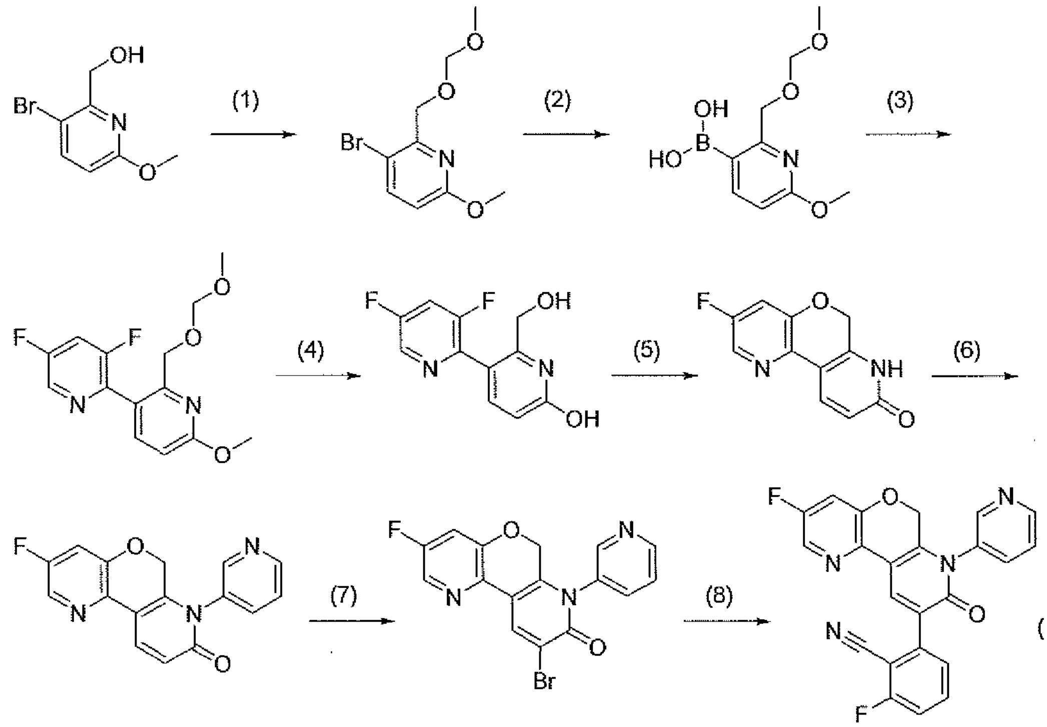

( I )

(1) Synthesis of 3-bromo-6-methoxy-2-((methoxymethoxy)methyl)pyridine

A mixture of (3-bromo-6-methoxypyridin-2-yl)methanol (CAS No. 623942-84-7)(2.15 g, 9.86 mmol, 1 equivalent), chloromethyl methyl ether (2.25 mL, 29.6 mmol, 3 equivalents) and N,N-diisopropylethylamine (8.61 mL, 49.3 mmol, 5 equivalents) was stirred in a DCM solution (45 mL) at room temperature for 15 hours. The reaction mixture was concentrated under reduced pressure. The residue was purified with silica gel column chromatography (NH silica gel, 0%-5% ethyl acetate/n-heptane) to afford the title compound (yield: 2.22 g).
MS $[M+H]^+$ = 262

(2) Synthesis of (6-methoxy-2-((methoxymethoxy)methyl)pyridin-3-yl)boronic acid

A solution of 3-bromo-6-methoxy-2-((methoxymethoxy)methyl)pyridine (1.09 g, 4.16 mmol, 1 equivalent) in THF (20 mL) was cooled to -78°C, and n-butyllithium (2.69 M solution in n-hexane, 1.70 mL, 4.58 mmol, 1.1 equivalents) was added. The reaction mixture was stirred at the same temperature for 1 hour, and then trimethyl borate (0.696 mL, 6.24 mmol, 1.5 equivalents) was added. The reaction mixture was stirred for 12 hours while warming up to room temperature, and then concentrated under reduced pressure. To the residue was added a saturated aqueous ammonium chloride solution, and the mixture was extracted with DCM. The organic layer was concentrated under reduced pressure, and the residue was purified with silica gel column chromatography (silica gel, 10%-100% ethyl acetate/n-heptane) to afford the title compound (yield: 570 mg).
$^1$H-NMR (400 MHz, $CDCl_3$) $\delta$ (ppm): 3.42 (s, 3H), 3.95 (s, 3H), 4.75 (s, 2H), 4.79 (s, 2H), 6.32 (s, 2H), 6.71 (d, J = 8.2 Hz, 1H), 8.08 (d, J = 8.4 Hz, 1H).
MS $[M+H]^+$ = 228

(3) Synthesis of 3,5-difluoro-6'-methoxy-2'-((methoxymethoxy)methyl)-2,3'-bipyridine

A mixture of (6-methoxy-2-((methoxymethoxy)methyl)pyridin-3-yl)boronic acid (570 mg, 2.51 mmol, 1 equivalent), 2-bromo-3,5-difluoropyridine (CAS No. 660425-16-1) (560 mg, 2.89 mmol, 1.15 equivalents), potassium fluoride (438 mg, 7.53 mmol, 3 equivalents), $(Ataphos)_2PdCl_2$ (89 mg, 0.126 mmol, 0.05 equivalents), 1,4-dioxane (12 mL) and water (3 mL) was reacted in a microwave reactor at 130°C for 4 hours. The reaction mixture was cooled down to room temperature, and then directly purified with silica gel column chromatography (silica gel on NH silica gel, 5%-35% ethyl acetate/n-heptane) to afford the title compound (yield: 648 mg).
MS $[M+H]^+$ = 297

(4) Synthesis of 3,5-difluoro-2'-(hydroxymethyl)-[2,3'-bipyridin]-6'-ol

A mixture of 3,5-difluoro-6'-methoxy-2'-((methoxymethoxy)methyl)-2,3'-bipyridine (648 mg, 2.19 mmol, 1 equivalent) and a 48% aqueous hydrobromic acid solution (1.98 mL, 17.5 mmol, 8 equivalents) was stirred in a THF (15 mL) solution at 55°C for 8 hours. The reaction mixture was cooled down to room temperature, and then concentrated under reduced pressure. To the residue were added a saturated aqueous sodium hydrogen carbonate solution and DCM. The mixture was stirred at room temperature for 1 hour, and then the resulting precipitate was collected by filtration. The resulting solid was washed with water to afford the title compound (yield: 349 mg).
$^1$H-NMR (400 MHz, DMSO-$d_6$) $\delta$ (ppm): 4.33 (s, 2H), 6.35 (d, J = 9.3 Hz, 1H), 7.51 (dd, J = 9.3, 1.8 Hz, 1H), 8.04 (ddd, J = 10.1, 8.9, 2.5 Hz, 1H), 8.57 (d, J = 2.4 Hz, 1H).
MS $[M+H]^+$ = 239

(5) Synthesis of 3-fluoro-6H-pyrano[3,2-b:5,4-b']dipyridin-8(7H)-one

A mixture of 3,5-difluoro-2'-(hydroxymethyl)-[2,3'-bipyridin]-6'-ol (329 mg, 1.38 mmol, 1 equivalent) and potassium carbonate (573 mg, 4.14 mmol, 3 equivalents) was stirred in a DMF (7 mL) solution at 100°C for 5 hours. The reaction mixture was cooled down to room temperature, and then an aqueous ammonium chloride solution was added. The mixture was stirred at room temperature for 30 minutes, and then the resulting precipitate was collected by filtration. The resulting solid was washed with water and n-heptane to afford the title compound (yield: 251 mg). $^1$H-NMR (400 MHz, DMSO-$d_6$) $\delta$ (ppm): 5.20 (s, 2H), 6.46 (d, J = 8.8 Hz, 1H), 7.38 (dd, J = 9.7, 2.4 Hz, 1H), 8.04 (d, J = 9.3 Hz, 1H), 8.19 (d, J = 2.6 Hz, 1H).
MS $[M+H]^+$ = 219

(6) Synthesis of 3-fluoro-7-(pyridin-3-yl)-6H-pyrano[3,2-b:5,4-b']dipyridin-8(7H)-one

A mixture of 3-fluoro-6H-pyrano[3,2-b:5,4-b']dipyridin-8(7H)-one (250 mg, 1.15 mmol, 1 equivalent), silver carbonate (474 mg, 1.72 mmol, 1.5 equivalents), copper(I) iodide (131 mg, 0.687 mmol, 0.6 equivalents) and pyridine (0.927 mL, 11.5 mmol, 10 equivalents) was stirred in a mixed solvent of DMF (7 mL) and DMSO (9 mL) at 80°C for 20 minutes. To the reaction mixture was slowly added a mixed solution of pyridine-3-boronic acid 1,3-propanediol cyclic ester (CAS No. 131534-65-1) (373 mg, 2.29 mmol, 2 equivalents) in DMF (4 mL) and DMSO (1 mL). The reaction mixture was stirred at 80°C for 20 hours, then applied to silica gel pad (NH silica gel and silica gel) and eluted with ethyl acetate. The resulting solution was concentrated under reduced pressure. To the residue was added water, and the mixture was extracted with ethyl acetate. The organic layer was washed with a saturated aqueous sodium chloride solution, and then concentrated under reduced pressure. The residue was purified with silica gel column chromatography (NH silica gel on silica gel, 10%-100% ethyl acetate/n-heptane, 10% methanol/ethyl acetate) to afford the title compound (yield: 43.5 mg).
$^1$H-NMR (400 MHz, $CDCl_3$) $\delta$ (ppm): 4.64-4.73 (m, 1H), 4.77-4.86 (m, 1H), 6.78 (d, J = 9.5 Hz, 1H), 6.94 (dd, J = 8.9, 2.5 Hz, 1H), 7.51-7.58 (m, 1H), 7.65-7.72 (m, 1H), 8.14 (d, J = 2.6 Hz, 1H), 8.29 (d, J = 9.7 Hz, 1H), 8.53 (d, J = 2.6 Hz, 1H), 8.79 (dd, J = 4.8, 1.5 Hz, 1H).
MS $[M+H]^+$ = 296

(7) Synthesis of 9-bromo-3-fluoro-7-(pyridin-3-yl)-6H-pyrano[3,2-b:5,4-b']dipyridin-8(7H)-one

A mixture of 3-fluoro-7-(pyridin-3-yl)-6H-pyrano[3,2-b:5,4-b']dipyridin-8(7H)-one (43.5 mg, 0.147 mmol, 1 equivalent) and NBS (31.5 mg, 0.177 mmol, 1.2 equivalents) was stirred in a acetonitrile solution (3 mL) at room temperature for 15 hours. The reaction mixture was directly purified with silica gel column chromatography (NH silica gel on silica gel, 10%-100% ethyl acetate/n-heptane) to afford the title compound (yield: 33 mg).
$^1$H-NMR (400 MHz, $CDCl_3$) $\delta$ (ppm): 4.60-4.70 (m, 1H), 4.74-4.83 (m, 1H), 6.95 (dd, J = 8.6, 2.0 Hz, 1H), 7.56 (dd, J = 8.1, 4.8 Hz, 1H), 7.65-7.72 (m, 1H), 8.14 (d, J = 2.4 Hz, 1H), 8.53 (d, J = 2.4 Hz, 1H), 8.71 (s, 1H), 8.81 (dd, J = 4.9, 1.6 Hz, 1H).
MS $[M+Na]^+$ = 396

(8) Synthesis of 2-fluoro-6-(3-fluoro-8-oxo-7-(pyridin-3-yl)-7,8-dihydro-6H-pyrano[3,2-b:5,4-b']dipyridin-9-yl)benzonitrile

A mixture of 9-bromo-3-fluoro-7-(pyridin-3-yl)-6H-pyrano[3,2-b:5,4-b']dipyridin-8(7H)-one (16.5 mg, 0.044 mmol, 1 equivalent), 2-cyano-3-fluorophenylboronic acid pinacol ester (CAS No. 765916-91-4) (14.2 mg, 0.057 mmol, 1.3 equivalents), $(Ataphos)_2PdCl_2$ (1.56 mg, 2.21 $\mu$mol, 0.05 equivalents), triethylamine (0.025 mL, 0.176 mmol, 4 equivalents), 1,4-dioxane (0.5 mL) and water (0.15 mL) was reacted in a microwave reactor at 130°C for 2.5 hours. The

reaction solution was directly purified with silica gel column chromatography (NH silica gel on silica gel, 5%-90% ethyl acetate/n-heptane) to afford the title compound (yield; 11.0 mg).
$^1$H-NMR (400 MHz, $CDCl_3$) $\delta$ (ppm): 4.71-4.80 (m, 1H), 4.86-4.95 (m, 1H), 6.97 (dd, J = 8.8, 2.4 Hz, 1H), 7.20-7.28 (m, 1H), 7.46 (d, J = 7.7 Hz, 1H), 7.57 (dd, J = 8.2, 4.8 Hz, 1H), 7.63 (td, J = 8.2, 5.8 Hz, 1H), 7.77 (ddd, J = 8.1, 2.6, 1.6 Hz, 1H), 8.14 (d, J = 2.6 Hz, 1H), 8.51 (s, 1H), 8.60 (d, J = 2.6 Hz, 1H), 8.81 (dd, J = 4.9, 1.6 Hz, 1H).
MS $[M+H]^+$ = 415

<Example 1>

Preparation of Type 1 anhydrate crystal of 2-fluoro-6-(3-fluoro-8-oxo-7-(pyridin-3-yl)-7,8-dihydro-6H-pyrano[3,2-b:5,4-b']dipyridin-9-yl)benzonitrile

3.2 g of 2-fluoro-6-(3-fluoro-8-oxo-7-(pyridin-3-yl)-7,8-dihydro-6H-pyrano[3,2-b:5,4-b']dipyridin-9-yl)benzonitrile described in Reference Example 1 was weighed into an eggplant flask, and 160 mL of 1-butanol was added thereto and the obtained solution was stirred with a stirrer to obtain a suspension. The obtained suspension was stirred at room temperature for 2 days. The resulting solid was filtered to afford a Type 1 crystal (yield: 2.8 g) of an anhydrate of the title compound. It should be noted that the Type 1 anhydrate crystal of this example is not obtained by starting from Type 3 anhydrate crystal of Example 2. Diffraction angles ($2\theta \pm 0.2°$) in X-ray powder diffraction: 5.1, 8.7, 10.2, 10.8, 11.6, 15.4, 16.8, 18.7, 19.6, 21.1, 23.4, 24.9, 26.2, 27.1, 28.1, 30.4, 31.0
$^{13}$C-NMR (100 MHz, solid state) $\delta$ (ppm): 65.8, 103.5, 107.2, 113.4, 114.7, 123.9, 124.7, 126.4, 131.6, 133.9, 135.6, 142.4, 145.1, 148.7, 152.9, 157.2, 160.1, 161.9

The obtained powder X-ray diffraction pattern is shown in Figure 1, and the obtained $^{13}$C solid state NMR spectrum is shown in Figure 2.

<Example 2>

Preparation of Type 3 anhydrate crystal of 2-fluoro-6-(3-fluoro-8-oxo-7-(pyridin-3-yl)-7,8-dihydro-6H-pyrano[3,2-b:5,4-b']dipyridin-9-yl)benzonitrile

2.2 g of 2-fluoro-6-(3-fluoro-8-oxo-7-(pyridin-3-yl)-7,8-dihydro-6H-pyrano[3,2-b:5,4-b']dipyridin-9-yl)benzonitrile described in Reference Example 1 was weighed into an eggplant flask, and 44 mL of 1-butanol was added thereto and the obtained solution was stirred with a stirrer to obtain a suspension. The obtained suspension was stirred at 40°C for 2 days, and then stirred at room temperature for 3 days. The resulting solid was filtered to afford a Type 3 crystal (yield: 2.1 g) of an anhydrate of the title compound.
Diffraction angles ($2\theta \pm 0.2°$) in X-ray powder diffraction: 11.8, 13.7, 15.8, 17.5, 18.4, 19.3, 21.1, 22.2, 23.6, 24.4, 25.5, 26.0, 27.0, 33.0
$^{13}$C-NMR (100 MHz, solid state) $\delta$ (ppm): 64.3, 103.1, 110.4, 111.9, 114.7, 122.7, 127.1, 131.5, 135.6, 142.0, 149.0, 150.5, 152.5, 157.3, 159.0, 161.5

The obtained powder X-ray diffraction pattern is shown in Figure 3, and the obtained $^{13}$C solid state NMR spectrum is shown in Figure 4.

<Example 3>

Preparation of hydrate crystal of 2-fluoro-6-(3-fluoro-8-oxo-7-(pyridin-3-yl)-7,8-dihydro-6H-pyrano[3,2-b:5,4-b']dipyridin-9-yl)benzonitrile

39 mg of 2-fluoro-6-(3-fluoro-8-oxo-7-(pyridin-3-yl)-7,8-dihydro-6H-pyrano[3,2-b:5,4-b']dipyridin-9-yl)benzonitrile described in Reference Example 1 was weighed into a test tube, and 50%(v/v) ethanol aqueous solution was added thereto and the obtained solution was stirred with a stirrer to obtain a suspension. Then, the obtained suspension was stirred at room temperature for 10 days. The resulting solid was filtered to afford the title compound.
Diffraction angles ($2\theta \pm 0.2°$) in X-ray powder diffraction: 5.5, 6.4, 8.5, 14.0, 15.4, 16.1, 17.0, 22.6, 25.8, 26.5, 27.2
$^{13}$C-NMR (100 MHz, solid state) $\delta$ (ppm): 63.7, 100.4, 103.5, 109.6, 111.8, 117.2, 125.3, 129.1, 134.0, 136.2, 142.4, 143.3, 146.1, 147.3, 149.5, 151.0, 153.0, 156.3, 159.3, 160.8, 161.7, 164.1

The obtained powder X-ray diffraction pattern is shown in Figure 5, and the obtained $^{13}$C solid state NMR spectrum is shown in Figure 6.

<Measurement of thermal analysis TG-DTA>

The crystal obtained in Example 3 (Hydrate crystal) was weighed precisely in an aluminum sample pan and measured under the following conditions. By heating, this hydrate crystal released water to be converted into an anhydrate crystal which was different from the Type 1 crystal and the Type 3 crystal. When returned to room temperature, the anhydrate crystal absorbed water and returned to the original hydrate crystal again.

(Measurement Conditions)

Atmosphere: under 40 mL/min nitrogen flow
Control: empty aluminum sample pan
Heating rate: 10°C/min
Sampling interval: 0.1 sec
Measurement temperature: room temperature to 230°C

The results are shown in Figure 7.

<Pharmacological Test Example>

With regard to the AMPA receptor inhibitory action of compound (I), AMPA-induced intraneuronal calcium entry-suppressing action was examined using a primary culture system of embryonic rat cerebral cortex neurons.

AMPA-Induced Intraneuronal Calcium Entry-Suppressing Action

(Culture Conditions)

Whole fetuses were removed from the uteri of Wistar rats at embryonic day 18 (CHARLES RIVER LABORATORIES JAPAN, INC.) anesthetized with isoflurane. Whole brains were removed from the whole fetuses, and the cerebral cortex was separated in Leibovitz's L-15 medium or Hanks balanced salt solution containing 20% fetal bovine serum. The resulting material was treated with trypsin/DNase solution at 37°C for 45 to 60 minutes. After the serum was added to stop the trypsin reaction, the resulting material was centrifuged at 1200 to 1500 rpm for 3 to 5 minutes. After the supernatant was removed, Neurobasal medium (containing 2% B-27 supplement and the like; hereinafter referred to as "Neurobasal medium") was added, and the cells were uniformly dispersed by gently pipetting. The cell suspension was slightly stirred, and then filtered through a nylon mesh filter. Viable cells were counted using a hemocytometer, and diluted with Neurobasal medium to 8 to 10 x $10^5$ cells/mL. The cells were seeded into 96 well plates at 100 μL per well and cultured in a $CO_2$ incubator (5% $CO_2$, 37°C). The medium was replaced after 24 hours of culturing, and neurons cultured for 7 to 21 days were used in the following pharmacological test.

On the day of the pharmacological test, the medium was replaced with a calcium measurement solution containing 5 to 10 μmol/L of Fura 2-AM (140 mmol/L sodium chloride, 5 mmol/L potassium chloride, 2 mmol/L magnesium chloride, 3 mmol/L calcium chloride, 24 mmol/L D(+)-glucose, 10 mmol/L HEPES, 1 μmol/L MK-801, pH 7.4), and the cells were treated in a $CO_2$ incubator (5% $CO_2$, 37°C) for 1 to 2 hours. The calcium measurement solution containing the Fura2-AM solution was subsequently removed, and the neurons in each well were washed twice with the calcium measurement solution, and then 50 μL of the calcium measurement solution was added to each well. Subsequently, 50 μL of a test compound or medium prepared at a concentration 3-fold higher than the final concentration was added, and the cells were pre-treated for about 15 minutes. The well plates were subsequently placed on the Fluorescence Drug Screening System 6000 (manufactured by Hamamatsu Photonics K.K.). The cells were stimulated by adding to each well 50 μL of an AMPA stimulating solution prepared to 3.3 μmol/L with the calcium measurement solution. Changes in calcium concentration were measured as the changes in fluorescence intensity at excitation wavelengths of 340 nm and 380 nm (measurement wavelength: 540 nm). The rate of change of the intracellular calcium concentration was calculated according to the following equation:

$$\text{Rate of increase of the intracellular calcium concentration (\% control)} = (T_{post} - T_{pre})/(C_{post} - C_{pre}) \times 100$$

wherein $T_{post}$: the fluorescence intensity of sample wells after stimulation;
$T_{pre}$: the fluorescence intensity of sample wells before stimulation; $C_{post}$: the fluorescence intensity of control wells after stimulation; and $C_{pre}$: the fluorescence intensity of control wells before stimulation.

The $IC_{50}$ value of the test compound for AMPA inhibition was determined from the rates of increase of the intracellular calcium concentration at various concentrations.

The results are shown in Table 1. These results revealed that compound (I) has sufficient AMPA receptor inhibitory action.

[Table 1]

| Sample | $IC_{50}$ (nM) |
|---|---|
| Compound (I) | 19.40 |

**Claims**

1. A crystal of 2-fluoro-6-(3-fluoro-8-oxo-7-(pyridin-3-yl)-7,8-dihydro-6H-pyrano[3,2-b:5,4-b']dipyridin-9-yl)benzonitrile represented by formula (I):

[Chemical Formula 1]

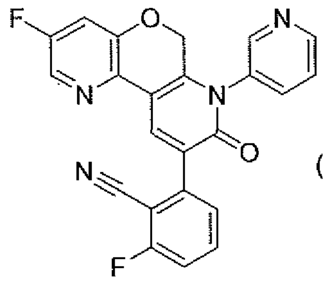

(I)

2. The crystal according to claim 1, wherein the crystal is a Type 1 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a diffraction peak at a diffraction angle (2θ ± 0.2°) of 10.8° in powder X-ray diffraction.

3. The crystal according to claim 2, wherein the crystal has diffraction peaks at diffraction angles (2θ ± 0.2°) of 10.2°, 10.8° and 30.4° in powder X-ray diffraction.

4. The crystal according to claim 1, wherein the crystal is a Type 1 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having peaks at chemical shifts (δ ± 0.5 ppm) of 65.8 ppm, 107.2 ppm, 113.4 ppm, 126.4 ppm, 145.1 ppm and 160.1 ppm in $^{13}C$ solid state NMR spectroscopy.

5. The crystal according to claim 1, wherein the crystal is a Type 1 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a powder X-ray diffraction pattern shown in Figure 1.

6. The crystal according to claim 1, wherein the crystal is a Type 3 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a diffraction peak at a diffraction angle (2θ ± 0.2°) of 24.4° in powder X-ray diffraction.

7. The crystal according to claim 6, wherein the crystal has diffraction peaks at diffraction angles (2θ ± 0.2°) of 15.8°, 18.4° and 24.4° in powder X-ray diffraction.

8. The crystal according to claim 1, wherein the crystal is a Type 3 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having peaks at chemical shifts (δ ± 0.5 ppm) of 64.3 ppm, 110.4 ppm, 122.7 ppm, and 127.1 ppm in $^{13}C$ solid state NMR spectroscopy.

9. The crystal according to claim 1, wherein the crystal is a Type 3 crystal of an anhydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a powder X-ray diffraction pattern shown in Figure 3.

**10.** The crystal according to claim 1, wherein the crystal is a crystal of a hydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a diffraction peak at a diffraction angle ($2\theta \pm 0.2°$) of 5.5° in powder X-ray diffraction.

**11.** The crystal according to claim 10, wherein the crystal has diffraction peaks at diffraction angles ($2\theta \pm 0.2°$) of 5.5°, 14.0° and 27.2° in powder X-ray diffraction.

**12.** The crystal according to claim 1, wherein the crystal is a crystal of a hydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having peaks at chemical shifts ($\delta \pm 0.5$ ppm) of 63.7 ppm, 100.4 ppm, 109.6 ppm, 117.2 ppm, 129.1 ppm, 147.3 ppm and 156.3 ppm in $^{13}C$ solid state NMR spectroscopy.

**13.** The crystal according to claim 1, wherein the crystal is a crystal of a hydrate of 2-fluoro-6-(3-fluoro-8-oxo-7-(pyridin-3-yl)-7,8-dihydro-6H-pyrano[3,2-b:5,4-b']dipyridin-9-yl)benzonitrile, having a powder X-ray diffraction pattern shown in Figure 5.

**14.** A pharmaceutical composition comprising the crystal according to any one of claims 1 to 13 as an active ingredient,

Fig.1

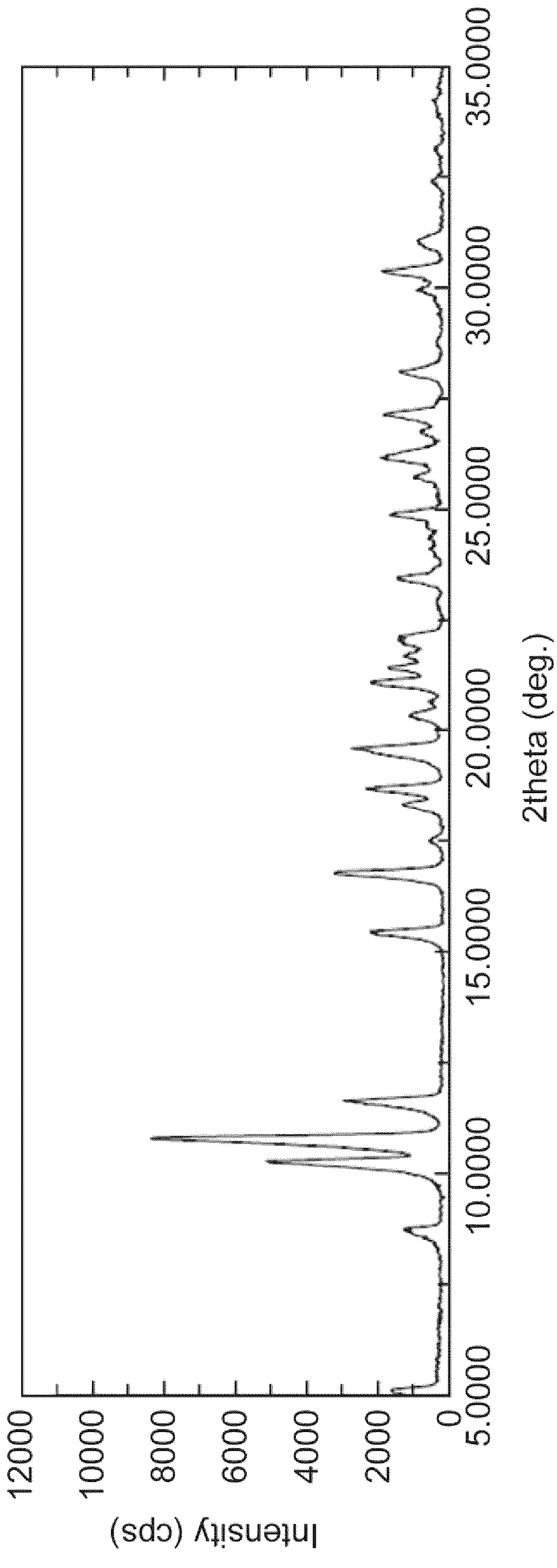
Intensity (cps)
12000
10000
8000
6000
4000
2000
0
5.0000
10.0000
15.0000
20.0000
25.0000
30.0000
35.0000
2theta (deg.)

Fig.2

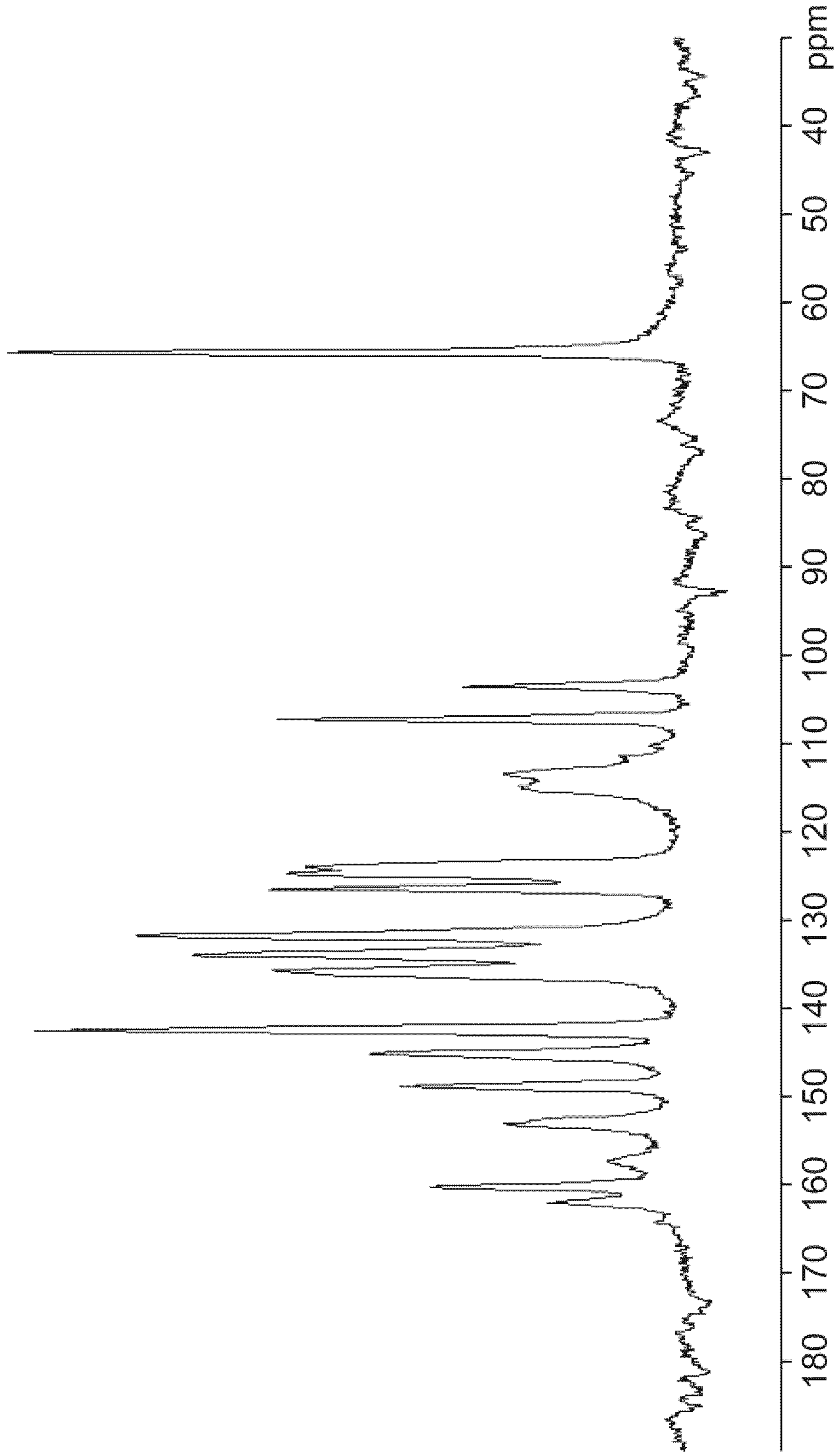

Fig.3

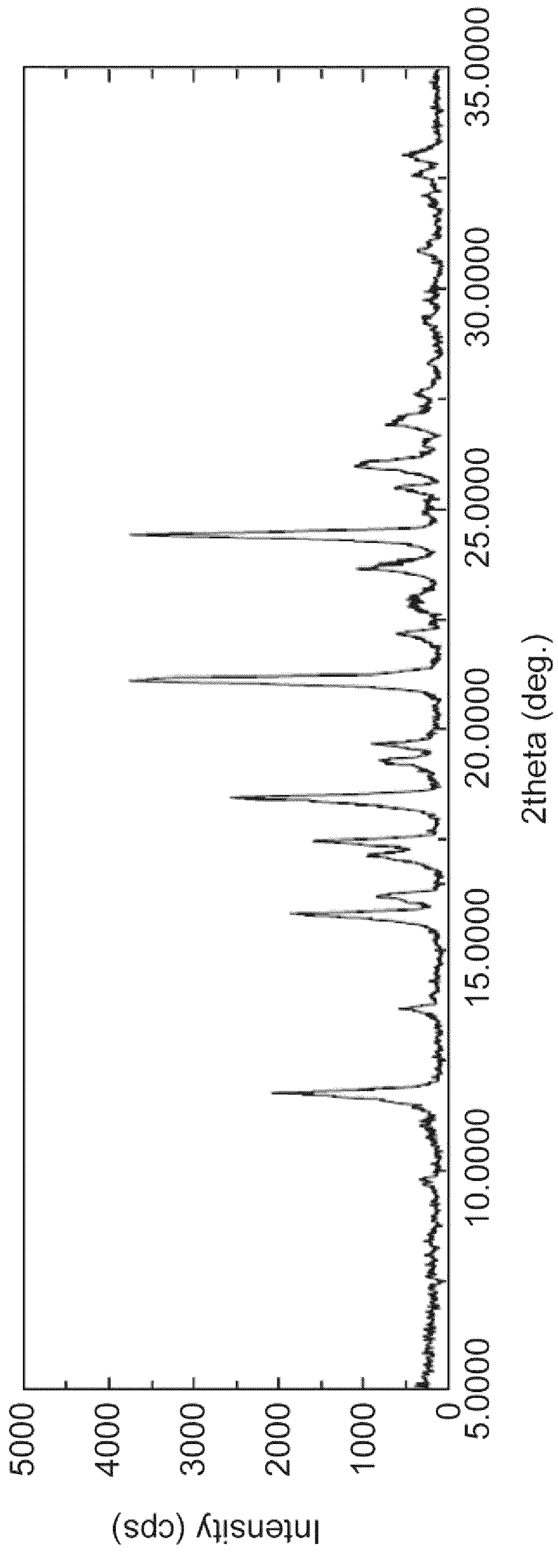
Intensity (cps)
5000
4000
3000
2000
1000
0
5.0000
10.0000
15.0000
20.0000
25.0000
30.0000
35.0000
2theta (deg.)

Fig.4

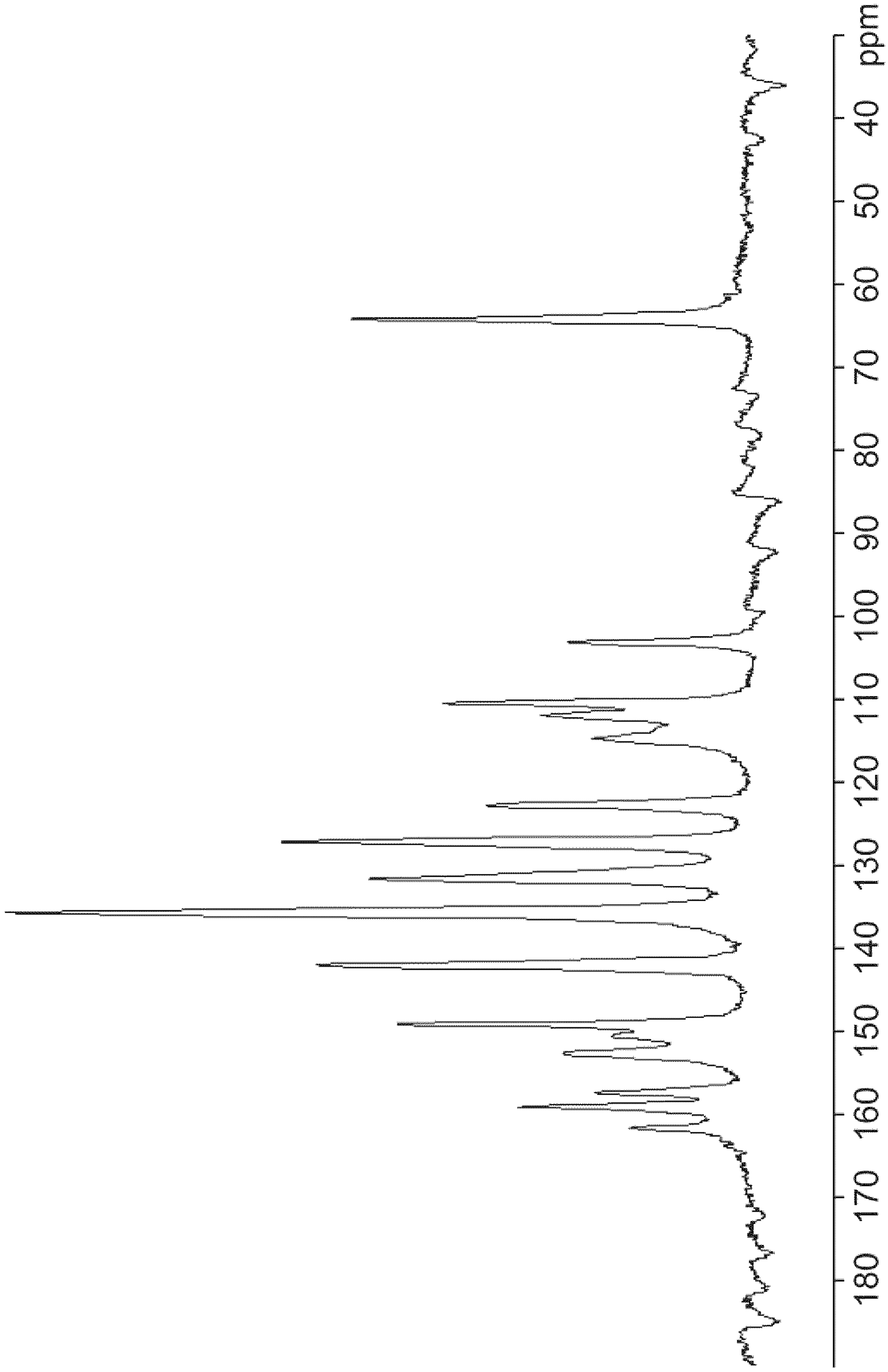

Fig.5

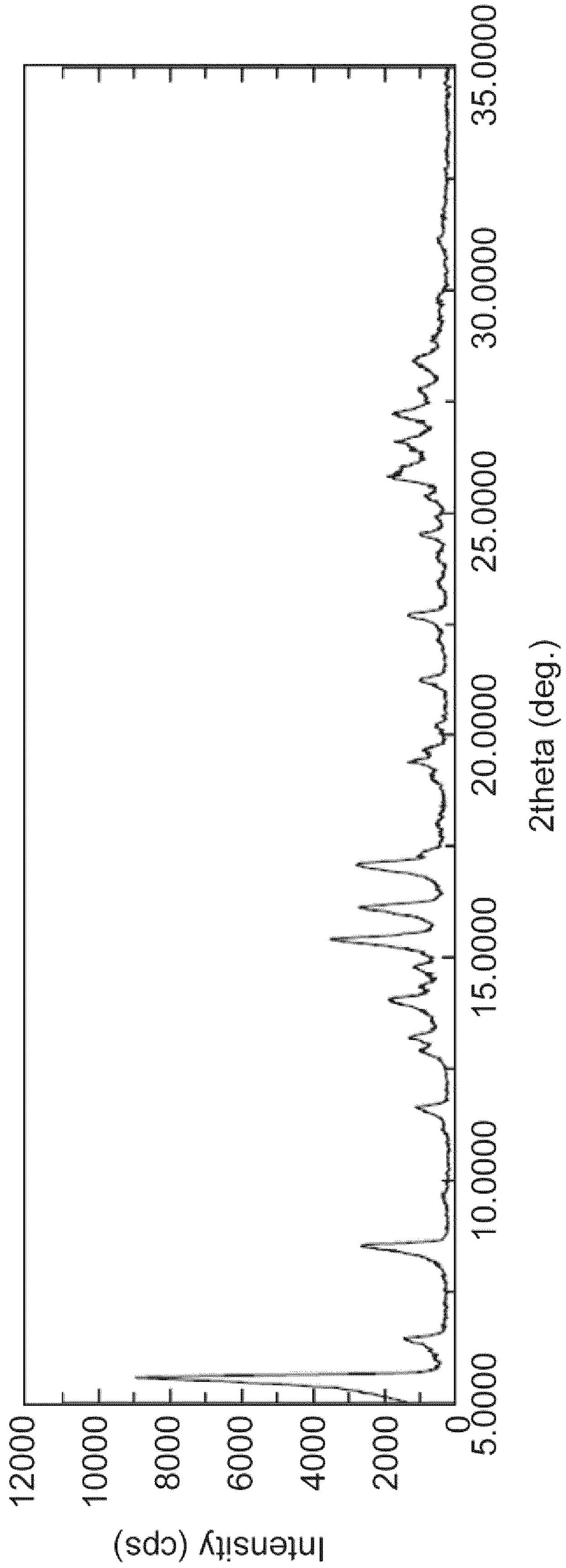
Intensity (cps)
12000
10000
8000
6000
4000
2000
0
5.0000
10.0000
15.0000
20.0000
25.0000
30.0000
35.0000
2theta (deg.)

Fig.6

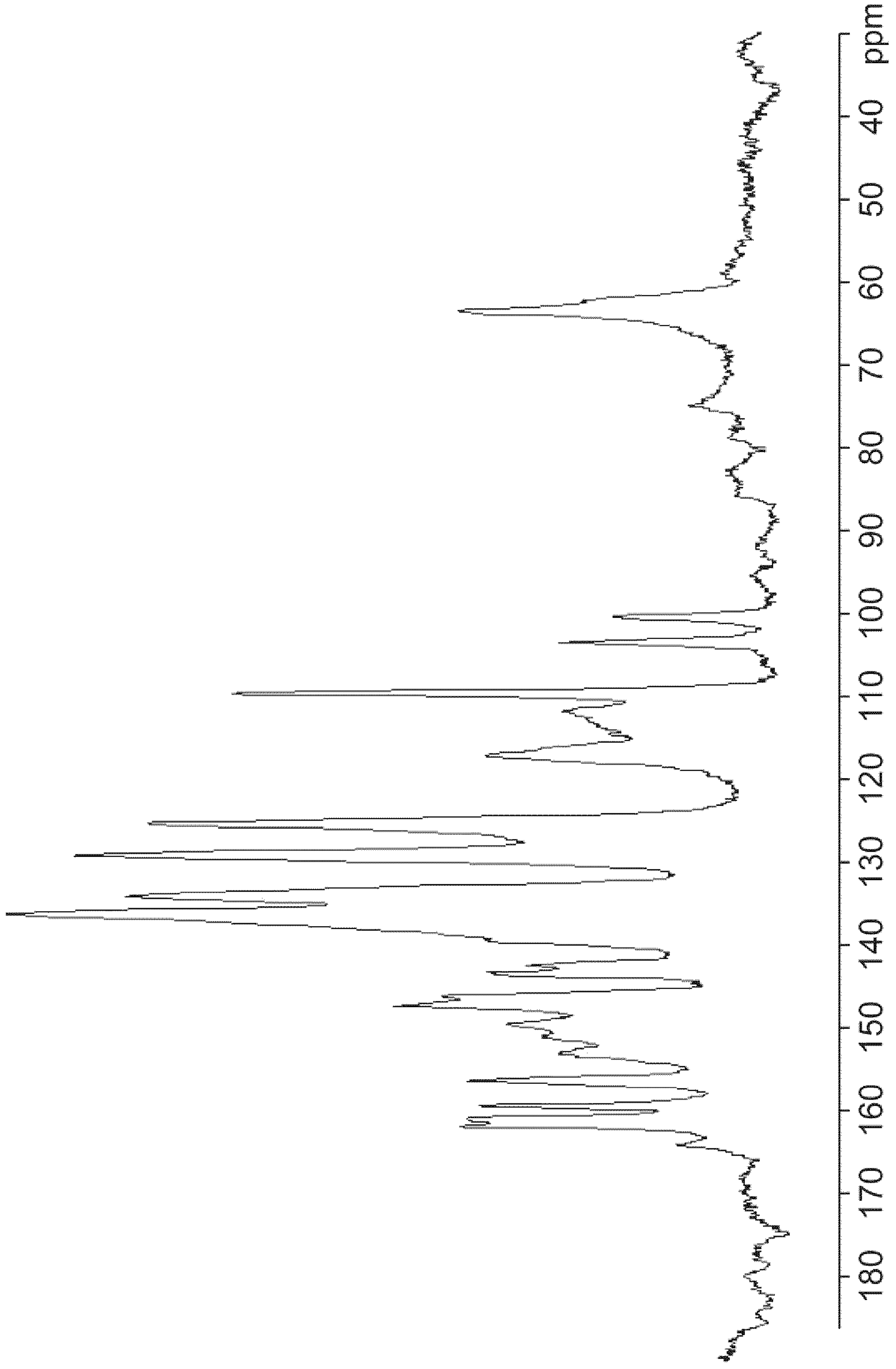

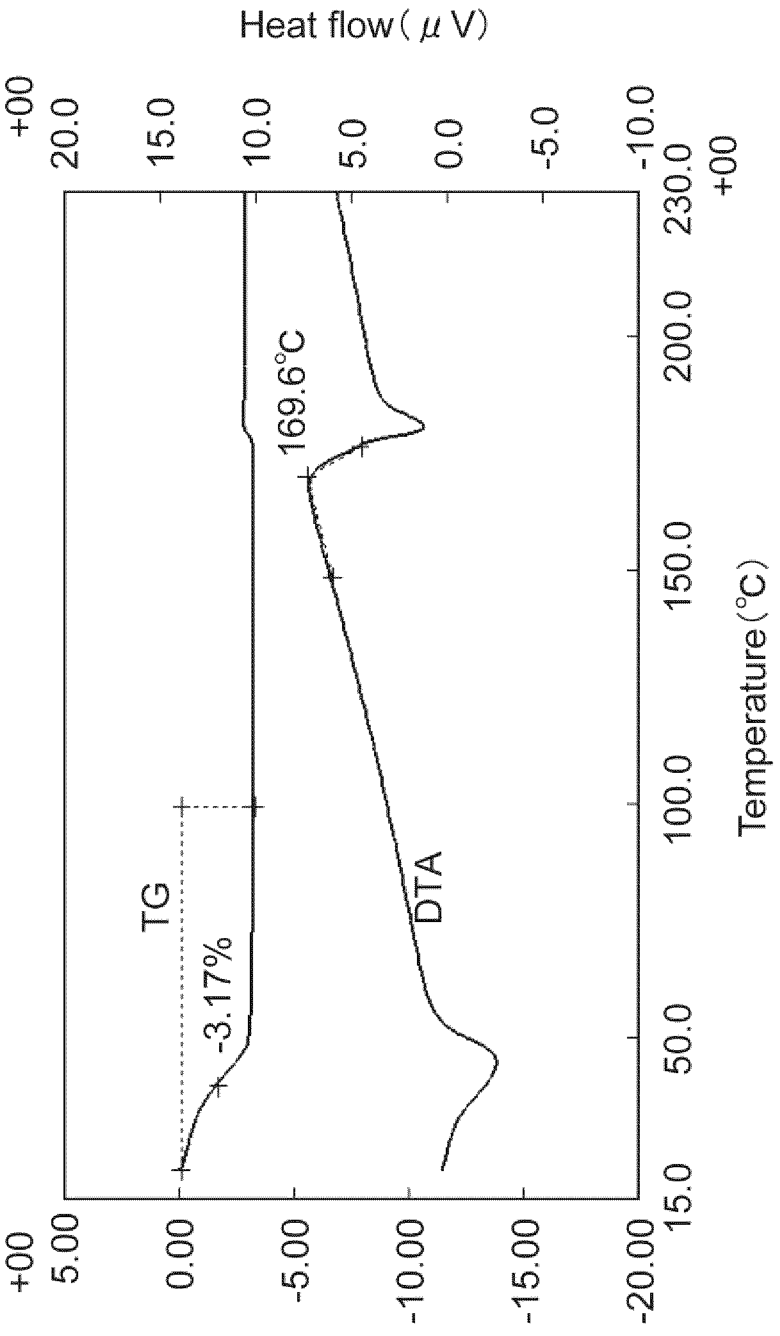
Heat flow ( μ V)
20.0
15.0
10.0
5.0
0.0
-5.0
-10.0
+00
169.6°C
TG
-3.17%
DTA
15.0
50.0
100.0
150.0
200.0
230.0
Temperature (°C)
5.00
0.00
-5.00
-10.00
-15.00
-20.00
Weight (%)

*Fig.7*

## INTERNATIONAL SEARCH REPORT

International application No.
PCT/JP2018/017577

A. CLASSIFICATION OF SUBJECT MATTER
Int.Cl. C07D491/147(2006.01)i, A61K31/444(2006.01)i, A61P25/08(2006.01)i

According to International Patent Classification (IPC) or to both national classification and IPC

B. FIELDS SEARCHED

Minimum documentation searched (classification system followed by classification symbols)
Int.Cl. C07D491/147, A61K31/444, A61P25/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2002/022587 A1 (EISAI CO., LTD.) 21 March 2002, entire text, in particular, see claims 1, 42, 50, examples 9, 54, table 1 & JP 2009-7356 A & JP 4233868 B2 & US 2003/0225081 A1, entire text, in particular, see claims 1, 42, 50, examples 9, 54, table 1 & US 2006/0189622 A1 & GB 102822 D0 & EP 1319659 A1 & KR 10-0770327 B1 & IL 154709 A & CN 1458924 A | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 July 2018 (06.07.2018) | 17 July 2018 (17.07.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/017577

C (Continuation). DOCUMENTS CONSIDERED TO BE RELEVANT

| Category* | Citation of document, with indication, where appropriate, of the relevant passages | Relevant to claim No. |
|---|---|---|
| A | JP 11-511451 A (BASF AG.) 05 October 1999, entire text, in particular, see claims 1, 3, 6, pp. 13, 14, examples 3, 15 & US 6407109 B1 & WO 1997/008168 A1, entire text, in particular, see claims 1, 3, 6, pp. 7-8, examples 3, 15 & EP 847395 A1 & DE 19532050 A & AU 6984696 A & CN 1194643 A | 1-14 |
| A | JP 52-062254 A (ABBOTT LABORATORIES) 23 May 1977, entire text, in particular, see claim 1, page 4, upper right column, example 1 & US 4018777 A, entire text, in particular, see claim 1, column 3, lines 33-35, example 1 & GB 1541858 A & DE 2651708 A & FR 2361887 A & FR 2397385 A & CH 618427 A & NL 7612607 A & NO 763881 A & AU 2114877 A & PH 12341 A & PT 65816 A & SE 7612620 A & FI 763241 A | 1-14 |
| A | HIBI, Shigeki et al., "Discovery of 2-(2-0xo-1-pheny 1-5-pyridin-2-y 1-1, 2-dihydropyridin-3-y1) ben zonitrile (Perampanel): A Novel, Noncompetitive a-Amino-3-hydroxy-5-methy 1-4-isoxazolepropanoic Acid (AMPA) Receptor Antagonist", J. MED. CHEM., 26 November 2012, vol. 55, pp. 10584-10600, entire text, in particular, see table 8, compound 28b | 1-14 |
| A | JP 2011-507799 A (EISAI R&D MANAGEMENT CO., LTD.) 10 March 2011, entire text, in particular, see claims 3, 4, compound A & US 2010/0256191 A1 & WO 2009/082038 A2, entire text, in particular, see claims 3, 4, compound A & EP 2222296 A2 | 1-14 |
| PA | WO 2017/082288 A1 (EISAI R&D MANAGEMENT CO., LTD.) 18 May 2017, entire text, in particular, see claims 8, 12-17, paragraphs [0013], [0020]-[0022], example 14, tables 1, 2, 4, fig. 2 & US 2017/0137436 A1, entire text, in particular, see claims 8, 12-19, paragraphs [0111], [0118]-[0120], example 14, tables 1, 2, 4, fig. 2 & TW 201720829 A & AR 106647 A | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0222587 A


### Non-patent literature cited in the description


- **DANIELA CATARZI et al.** *Medicinal Research Reviews,* 2007, vol. 27 (2), 239-278
- **ROGAWSKI MA et al.** *Acta Neurol Scand Suppl.,* 2013, vol. 197, 9-18
- **ALFONSO TORTORELLA et al.** *JPET,* 1997, vol. 280, 1401-1405
- **DE SARRO et al.** *Curr Top Med Chem.,* 2005, vol. 5 (1), 31-42
- **RUSSO E et al.** *Expert Opin Investig Drugs.,* September 2012, vol. 21 (9), 1371-89
- **BRITA FRITSCH et al.** *Epilepsia,* 2010, vol. 51 (1), 108-117
- **HOSFORD DA et al.** *J Neurosci,* 1991, vol. 11, 428-434
- **SHUN-ICHI YAMAGUCHI et al.** *Epilepsy Research,* 1993, vol. 15, 179-184
- **HANADA T.** *Expert Opin Drug Discov.,* 24 February 2014, vol. 9 (4), 449-458
- General Rules for Preparations of the Japanese Pharmacopoeia
- *CHEMICAL ABSTRACTS,* 623942-84-7
- *CHEMICAL ABSTRACTS,* 660425-16-1
- *CHEMICAL ABSTRACTS,* 131534-65-1
- *CHEMICAL ABSTRACTS,* 765916-91-4